# EUROPEAN PATENT SPECIFICATION

(11) **EP 2 353 648 B1**
(45) Date of publication and mention of the grant of the patent: **30.03.2016**
(21) Application number: 11000504.8
(22) Date of filing: 21.01.2011
(51) Int. Cl.: A61N 5/10

(54) **Particle beam treatment apparatus and irradiation nozzle apparatus**
Teilchenstrahlbehandlungsvorrichtung und Bestrahlungskopfvorrichtung
Appareil de traitement de faisceau à particules et appareil à buse d'irradiation

(30) Priority: 28.01.2010 JP 2010016945
(43) Date of publication of application: 10.08.2011
(73) Proprietor: Hitachi, Ltd., Chiyoda-ku Tokyo 100-8280 (JP)
(72) Inventor: Shinagawa, Ryosuke, Chiyoda-ku Tokyo 100-8220 (JP); Matsuda, Koji, Chiyoda-ku Tokyo 100-8220 (JP); Nakano, Tatsuya, Chiyoda-ku Tokyo 100-8220 (JP)
(74) Representative: Strehl Schübel-Hopf & Partner

(56) References cited:
- EP-A1- 1 479 411
- JP-A- 2007 268 035
- US-A1- 2006 067 468
- US-A1- 2006 113 497

## Description

### BACKGROUND OF THE INVENTION

### 1. Field of the Invention

The present invention relates to a particle beam treatment apparatus and an irradiation nozzle apparatus which irradiate an object (patient) with a charged particle beam such as protons or carbon ions.

### 2. Description of the Related Art

A particle beam treatment apparatus is known as an apparatus that treats cancer and the like. Such a particle beam treatment apparatus is configured to irradiate an affected part of a patient with charged particle beams (hereinafter also referred to as beam) such as protons or carbon ions.

The particle beam treatment apparatus includes a beam generator, a beam transport device and an irradiated field forming apparatus (hereinafter referred to as irradiation nozzle apparatus). The beam generator accelerates a beam that travels on a circular trajectory until the beam has predetermined energy. The beam having been accelerated to the predetermined energy level is transported through the beam transport device to the irradiation nozzle apparatus and extracted from the irradiation nozzle apparatus. Then, the irradiation nozzle apparatus irradiates with the extracted beam an affected part of a patient in a treatment room.

One of irradiation methods using a particle beam treatment apparatus is a beam scanning irradiation method described in Japanese Patent No. 2833602. In this irradiation method, an affected part of a patient is treated as multiple layers arranged in the depth direction (in which a beam propagates) of the body of the patient from the surface of the body; and each of the multiple layers is irradiated with a beam. A pair of scanning magnets is provided in the irradiation nozzle apparatus. The scanning magnets deflect a fine beam so that the beam is scanned over a range of irradiation. When the energy of the beam is changed, a layer to be irradiated with the beam is changed to another layer. When the amounts of currents to excite the scanning magnets are changed, the position of a point (of a surface substantially perpendicular to the direction in which the beam propagates) that is irradiated with the beam is changed. The thus-obtained beam scanning provides the irradiated field necessary for the patient.

The beam scanning irradiation method does not use a device such as a collimator that is necessary when another irradiation method is applied. This is advantageous in that the number of devices to be arranged on a beam path in an irradiation apparatus can be reduced. By contrast, it is essential, in the beam scanning irradiation method, to enhance the accuracy in positioning of the affected part to be irradiated with the beam. In addition, the advantage of the beam scanning irradiation method is that, as the beam has a smaller diameter, it is possible to irradiate edge parts of a field to be irradiated with the beam in a finer manner (lateral penumbra is small). This leads to suppressing undesired irradiation to which normal tissues are subject.

When air is present in the beam path located in the irradiation apparatus, the beam may be scattered by the air. Thus, there is a limitation on a reduction in the diameter of the beam. To overcome such inconvenience, JP-2007-268035-A discloses that a beam transport chamber is arranged within an irradiation nozzle apparatus. This beam transport chamber is made of metal or fiber-reinforced resin. The beam transport chamber is filled with gas (such as helium) that suppresses scattering (caused by air) of a beam. Alternatively the beam transport chamber has a vacuum region used to suppress scattering (caused by air) of the beam.

One of important processes in particle beam treatment is to position a patient. Positioning of a patient is performed by comparing digitally reconstructed radiograph (DRR) information with plain X-ray image information as described in JP-2006-239403-A. The digitally reconstructed radiograph (DRR) information is generated on the basis of a computed tomography (CT) image when a treatment plan is created. The plain X-ray image information is obtained by an X-ray apparatus from the patient lying on a treatment bed before irradiation with a beam during treatment. The X-ray apparatus includes an X-ray generator (X-ray tube) and an X-ray detector. The X-ray generator is located in an irradiation nozzle apparatus. The X-ray detector is located outside the irradiation nozzle apparatus. In addition, the X-ray detector is located on the opposite side of the irradiation nozzle apparatus with respect to a couch.

In addition, United States Patent Application No. 2004/0024300 A1 discloses a method of providing two X-ray tubes outside an irradiation nozzle apparatus in an X-ray treatment apparatus and emitting X-ray beams for positioning at different angles from each of the two X-ray tubes.

### SUMMARY OF THE INVENTION

When the beam transport chamber is arranged in the irradiation nozzle apparatus as described in JP-2007-268035-A, the effect of suppressing scattering (caused by air) of the beam is increased as the beam transport chamber is elongated; therefore it is possible to reduce the diameter of the beam in such a beam scanning irradiation method as described in Japanese Patent No. 2833602. However, the treatment apparatus described in JP-2006-239403-A includes the X-ray tube located in the irradiation nozzle apparatus to position the patient using X-ray image information, with the X-ray tube being large and heavy. In addition, the optimum length of the irradiation nozzle apparatus is determined on the basis of a space in a rotating gantry. Thus, when the beam transport chamber is arranged in the irradiation nozzle apparatus described in JP-2006-239403-A, the X-ray tube restricts a space in the irradiation nozzle apparatus where the beam transport chamber is arranged; therefore the beam transport chamber cannot be arranged over a specific length or more. As a result, an attempt to reduce the diameter of a beam is limited.

For the treatment apparatus described in United States Patent Application 2004/0024300 A1, a mechanism for a rotating gantry is doubled up, the treatment apparatus is mechanically complicated, and its overall cost is increased. In addition, the treatment apparatus described in United States Patent Application 2004/0024300 A1 is an X-ray treatment apparatus. Thus, when the technique based on United States Patent Application 2004/0024300 A1 is introduced in a proton treatment apparatus as it is, the proton treatment apparatus will disadvantageously have a limited imaging range. The reason that the imaging range is limited is that an irradiation nozzle apparatus for the proton treatment apparatus is larger than that for the X-ray treatment apparatus.

An object of the present invention is to provide, for obtaining a fine beam in a beam scanning irradiation method, a particle beam treatment apparatus and an irradiation nozzle apparatus which is adapted to ensure a space for installation of a beam transport chamber in the irradiation nozzle apparatus, suppress an increase in the cost of the particle beam treatment apparatus, and ensure a necessary imaging range.

To accomplish the aforementioned object, according to the present invention, an X-ray tube is located outside a scanning type irradiation nozzle apparatus that includes scanning magnets, whereas an X-ray detector is located inside the scanning type irradiation nozzle apparatus. In a conventional structure, an X-ray tube is located inside an irradiation nozzle apparatus and an X-ray detector is located outside that. According to the present invention, the X-ray detector, especially that including a flat panel detector is smaller in thickness in the direction of a beam axis and is simpler in structure than that of the X-ray tube. This makes it possible to ensure a space for installation of the beam transport chamber in the irradiation nozzle apparatus, and increase the length of the beam transport chamber in the apparatus compared with the conventional structure. It is, therefore, possible to suppress scattering of the beam caused by air during transport of the beam and reduce the diameter of the beam compared with the conventional structure.

In addition, since the X-ray detector is smaller in thickness in the direction of the beam axis and is simpler in structure than that of the X-ray tube, high degree of freedom as to where to arrange the X-ray detector is provided. Thus, the X-ray tube can be installed in a position shifted to the downstream side of the irradiation nozzle apparatus compared with a position at which a conventional X-ray tube is located. For example, the scanning type irradiation nozzle apparatus may include a beam monitor (e.g., beam dose monitor) arranged in a position on the downstream side of the scanning magnets and the beam transport chamber with respect to the direction in which the beam propagates. In this case, the X-ray detector may be installed in a position on a further downstream side relative to the beam monitor. This makes it possible to ensure a space for installation of the beam transport chamber in such a manner that the beam transport chamber can be located at the optimum position in the irradiation nozzle apparatus. Furthermore, it is possible to increase the length of the beam transport chamber, thereby reducing the diameter of the beam compared with the conventional structure.

In addition, only replacing the positions of the X-ray tube and the X-ray detector with each other is performed, thereby making it possible to suppress an increase in the cost of the particle beam treatment apparatus and ensure a necessary imaging range.

According to the present invention, it is possible to ensure a space for installation of the beam transport chamber in the irradiation nozzle apparatus. As a result, while the length of the irradiation nozzle apparatus is as long as that of the conventional one, the irradiation nozzle apparatus may include the beam transport chamber whose length is longer than the conventional structure. Thus, it is possible to reduce the diameter of the beam compared with the conventional structure. In addition, it is possible to suppress an increase in the cost of the particle beam treatment apparatus and ensure a necessary imaging range.

### BRIEF DESCRIPTION OF THE DRAWINGS

Fig. 1 is a diagram showing the entire configuration of a particle beam treatment apparatus according to an embodiment of the present invention.
Fig. 2 is a diagram showing the configuration of the inside of a rotating gantry when the rotating gantry is viewed from the side of a treatment room.
Fig. 3 is a diagram showing the configuration of a scanning type irradiation nozzle apparatus.
Fig. 4 is a diagram showing the state in which an X-ray tube and an X-ray detector are retracted from the axis of a beam in the case where the beam propagates from an upper side in a vertical direction.
Fig. 5 is a diagram showing, as a comparative example, the configuration of a conventional scanning type irradiation nozzle apparatus that includes an X-ray tube and a beam transport chamber.
Fig. 6 is a diagram showing a scanning type irradiation nozzle apparatus according to another embodiment of the present invention, while the irradiation nozzle apparatus is similar to that shown in Fig. 3 and includes a beam transport chamber that has a vacuum region instead of a gas region.

### DESCRIPTION OF THE PREFERRED EMBODIMENTS

A particle beam treatment apparatus according to a preferred embodiment of the present invention is described with referenced to the accompanying drawings.

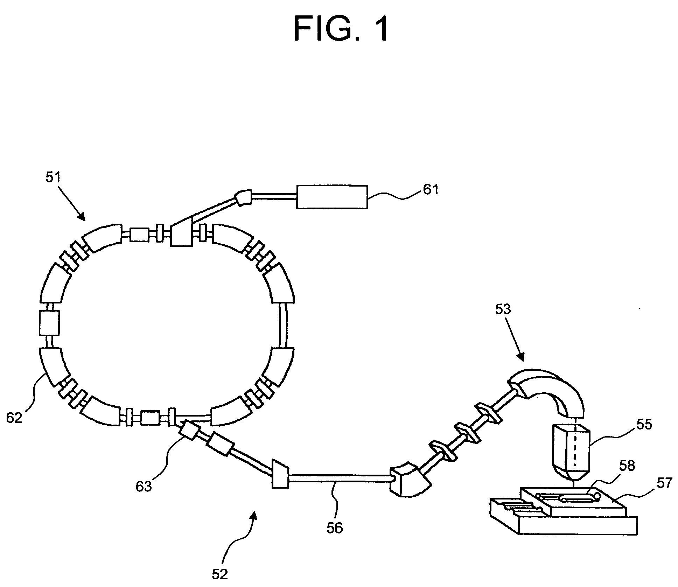

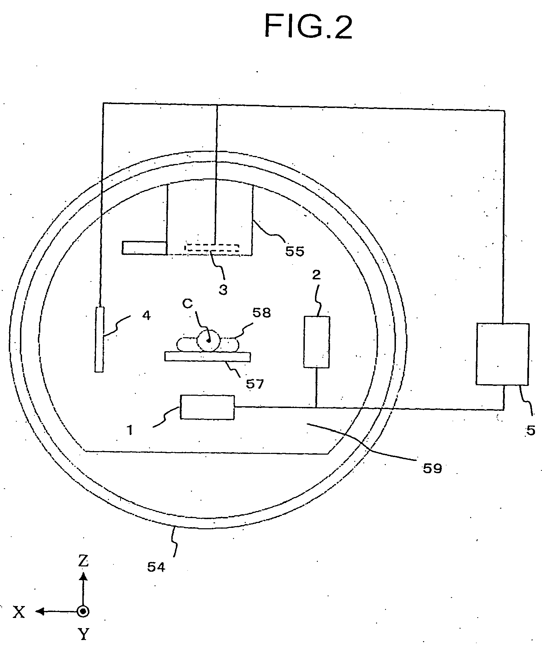

Fig. 1 is an outline diagram showing the entire configuration of the particle treatment apparatus according to the embodiment of the present invention. The particle treatment apparatus includes a charged particle beam generator 51, a beam transport unit 52 and a rotary irradiation apparatus 53. The rotary irradiation apparatus 53 includes a rotating gantry 54 and a scanning type irradiation nozzle apparatus 55. The rotating gantry 54 (refer to Fig. 2) is located in a treatment room. The scanning type irradiation nozzle apparatus 55 is attached to the rotating gantry 54.

The charged particle beam generator 51 includes an ion source (not shown), an upstream accelerator or injector (e.g., linear accelerator) 61 and a synchrotron 62. The ions (e.g., proton ions (or carbon ions)) generated by the ion source are accelerated by the upstream accelerator or injector 61 so as to form an ion beam, and then, the ion beam is extracted from the upstream accelerator 61 to propagate into the synchrotron 62. After the ion beam is accelerated to a set energy level by the synchrotron 62, the ion beam is extracted from the synchrotron 62 through an extraction deflector 63. Then, the ion beam extracted reaches the irradiation nozzle apparatus 55 included in the rotary irradiation apparatus 53, through a beam transport duct 56. Then, the ion beam propagates from the irradiation nozzle apparatus 55 to an affected part of a patient 58 lying on a couch (sheet or bed) 57 so that the affected part of the patient 58 is irradiated with the ion beam. A part of the beam transport duct 56 is attached to the rotating gantry 54 included in the rotary irradiation apparatus 53 and rotates together with the rotating gantry 54.

Fig. 2 is a diagram showing the configuration of the inside of the rotating gantry 54 when the rotating gantry 54 is viewed from the opposite side (back side of the rotating gantry) of the treatment room.

The rotating gantry 54 is held by a plurality of support rollers (not shown) and can rotate. The scanning type irradiation nozzle apparatus 55 is attached to a rotary body of the rotating gantry 54 and rotates together with the rotating gantry 54. In the rotating gantry 54, an irradiation room 59 is provided with the couch 57 arranged therein. By rotating the rotating gantry 54, the orientation of the irradiation nozzle apparatus 55 is changed so as to circle around the patient 58. The couch 57 is capable of three-dimensionally moving in X, Y and Z axial directions, and is also capable of rotating around X, Y and Z axes. Since the rotating gantry 54 is capable of rotating and the couch 57 is capable of moving and rotating in the aforementioned manner, the patient 58 can be irradiated from any direction. Y axis is set parallel to the rotational axis of the rotating gantry 54, while X axis is set perpendicular to the rotational axis of the rotating gantry 54 and parallel to a horizontal direction. Z axis is set perpendicular to the rotational axis of the rotating gantry 54 and parallel to the vertical direction.

In the rotating gantry 54, X-ray tubes (X-ray generators) 1 and 2 and X-ray detectors 3 and 4 are arranged in order to obtain X-ray image information that is used for positioning of the patient 58 (or positioning of the couch 57). The X-ray tube 1 faces the X-ray detector 3, while the patient 58 (couch 57) is located between the X-ray tube 1 and the X-ray detector 3. An X-ray beam emitted by the X-ray tube 1 passes through the patient 58 and is detected by the X-ray detector 3. The X-ray tube 1 and the X-ray detector 3 constitute a first X-ray imaging device. The X-ray detector 3 is arranged in the irradiation nozzle apparatus 55 (described later). The X-ray tube 2 faces the X-ray detector 4, while the patient 58 (couch 57) is located between the X-ray tube 2 and the X-ray detector 4, in a direction perpendicular to a direction in which the X-ray tube 1 and the X-ray detector 3 face each other. An X-ray beam emitted by the X-ray tube 2 passes through the patient 58 and is detected by the X-ray detector 4. The X-ray tube 2 and the X-ray detector 4 constitute a second X-ray imaging device. A pair of the X-ray tube 1 and the X-ray detector 3 and a pair of the X-ray tube 2 and the X-ray detector 4 rotate together with the rotating gantry 54.

The X-ray tubes 1 and 2 and the X-ray detectors 3 and 4 are connected to an X-ray imaging control device 5. The X-ray tubes 1 and 2 are driven by the X-ray imaging control device 5. The X-ray detectors 3 and 4 acquire X-ray information (pulse signals) and transmit the information to the X-ray imaging control device 5 for generation of X-ray image information on the basis of the received X-ray information.

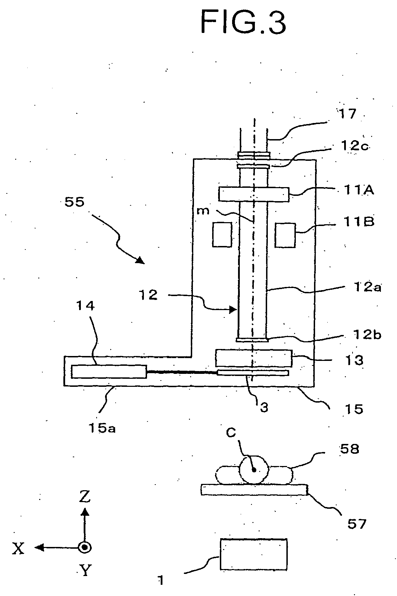

Fig. 3 is a diagram showing the configuration of the scanning type irradiation nozzle apparatus viewed from the opposite side (back side of the rotating gantry) of the treatment room.

Referring to Fig. 3, the scanning type irradiation nozzle apparatus 55 includes an X-direction beam scanning magnet 11A, a Y-direction beam scanning magnet 11B, a beam transport chamber 12, a beam monitor 13, the X-ray detector (including a flat panel detector) 3, an X-ray detector driving actuator 14 and a housing 15 that is made of metal. The X-direction beam scanning magnet 11A, the Y-direction beam scanning magnet 11B, the beam transport chamber 12, the beam monitor 13, the X-ray detector (including the flat panel detector) 3, the X-ray detector driving actuator 14 are arranged in the housing 15.

The X-direction beam scanning magnet 11A and the Y-direction beam scanning magnet 11B deflect a charged particle beam introduced into the irradiation nozzle apparatus 55, so that the affected part of the patient 58 is scanned with the charged particle beam during an irradiation process and an irradiated field that extends in the horizontal direction is formed.

The beam transport chamber 12 is a device that has a gas region or a vacuum region to suppress scattering of the charged particle beam. The beam transport chamber 12 includes: a chamber container 12a that has a rectangular shape in vertical cross section and is made of ceramic or fiber-reinforced resin; and isolated windows 12b and 12c that close both ends of the chamber container 12a. The beam transport chamber 12 is hollow inside. In the present embodiment, the beam transport chamber 12 is filled with helium gas so as to have a helium region. The isolated window 12c is located at the upper-side end of the beam transport chamber 12 so as to face a beam duct 17 that is a part of the beam transport duct 56 and to include a beam axis m therein, in the housing 15. The beam axis m is a central trajectory of the beam. The particle beam treatment apparatus is designed so that the beam axis m passes through a designed beam trajectory of the beam transport unit 52 and an iso-center (irradiation center) C located on the rotational axis of the rotating gantry 54.

The beam monitor 13 is a beam dose monitor that detects the dose of the charged particle beam with which the affected part of the patient is irradiated, for example. The beam monitor 13 may include a beam position monitor which detects the irradiation position and width of (a spot of) the charged particle beam on the affected part of the patient. The beam monitor 13 is arranged in the housing and located on the downstream side of the scanning magnets 11A and 11B and the beam transport chamber 12 with respect to the direction in which the beam propagates.

The X-ray detector 3 includes, for example, the flat panel detector, and is located on the downstream side of the beam dose monitor 13 with respect to the direction in which the beam propagates, so as to be retractable from the beam axis m in a rotational direction (X direction in Fig. 3) of the rotating gantry 54, in the housing 15.

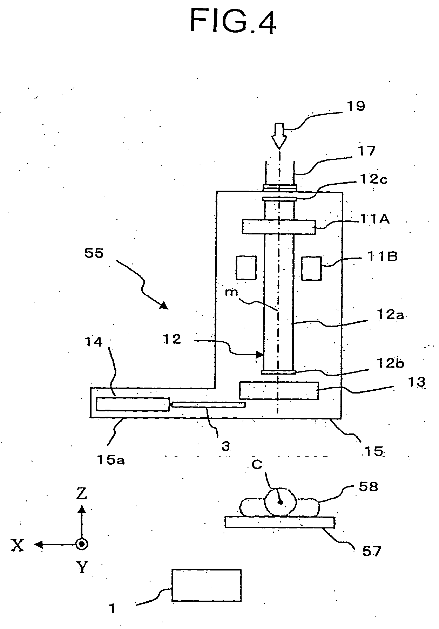

The X-ray detector driving actuator 14 is a double-acting air cylinder, for example. When air is supplied into a bottom-side cylinder room of the X-ray detector driving actuator 14, the X-ray detector driving actuator 14 elongates and thereby presses the X-ray detector 3 so that the X-ray detector 3 is located at an inserted position (shown in Fig. 3). When air is supplied into a rod-side cylinder room of the X-ray detector driving actuator 14, the X-ray detector driving actuator 14 shrinks and thereby pulls the X-ray detector 3 so that the X-ray detector 3 is located at a retracted position (shown in Fig. 4). The housing 15 has a projecting portion 15a in which the air cylinder 14 is located as shown in Figs. 3 and 4.

In Figs. 3 and 4, the air cylinder 14 is arranged on the left side (toward which the rotating gantry 54 rotates) of the irradiation nozzle apparatus 55 in consideration of a home position of the rotating gantry 54. The home position of the rotating gantry 54 is obtained by rotating 270 degrees from the state shown in Fig. 2 in the counterclockwise direction in Fig. 2. When the rotating gantry 54 is located at the home position, the irradiation nozzle apparatus 55 is located near a floor surface of the irradiation room 59 that is located on the right side of Fig. 2. Thus, a doctor or the like can easily reach the irradiation nozzle apparatus 55. Further, since the projecting portion 15a is located on the left side of the irradiation nozzle apparatus 55 as shown in Fig. 3, when the rotating gantry 54 is located at the home position, the projection 15a extends upward and does not reduce the range of activities of an operator such as a doctor.

Next, operations of the particle beam treatment apparatus during positioning of the patient and beam irradiation are described. In the following description, operations of devices of the particle beam treatment apparatus are each started according to an instruction provided by an operating device (not shown) used by the operator such as a doctor.

### Positioning of patient

During positioning of the patient 58, the rotating gantry 54 is rotated by 90 degrees from the home position in the counterclockwise direction so that the rotating gantry 54 is in the state shown in Fig. 2. In the state shown in Fig. 2, the X-ray tube 1 of the first X-ray imaging device is located directly under the patient 58 in the vertical direction; the X-ray detector 3 is located directly above the patient 58; the X-ray tube 2 is located on the right side of the patient 58 so that the X-ray tube 2 and the patient 58 are located side by side in the horizontal direction; and the X-ray detector 4 is located on the left side of the patient 58 so that the X-ray detector 4 and the patient 58 are located side by side in the horizontal direction.

Then, air is supplied into the bottom-side cylinder of the air cylinder 14 so that the X-ray detector 3 of the first X-ray imaging device is pressed by the air cylinder 14 and positioned at the inserted position shown in Fig. 3.

Then, the operator such as a doctor provides an instruction to start imaging. According to the instruction, the X-ray tube 1 of the first X-ray imaging device operates and emits an X-ray beam that is X-ray information. The X-ray information that passes through the patient 58 is detected by the X-ray detector 3. The X-ray detector 3 outputs the detected X-ray information to the X-ray imaging control device 5. The X-ray imaging control device 5 receives the X-ray information and generates X-ray image information on the basis of the received X-ray information. Then, the X-ray tube 2 of the second X-ray imaging device operates and emits an X-ray beam that is X-ray information. The X-ray information that passes through the patient 58 is detected by the X-ray detector 4. The X-ray detector 4 outputs the detected X-ray information to the X-ray imaging control device 5. The X-ray imaging control device 5 receives the X-ray information and generates X-ray image information on the basis of the received X-ray information.

The X-ray imaging control device 5 transmits the X-ray image information (obtained from the two directions) to a positioning control device (not shown). The positioning control device compares the X-ray image information obtained from the two directions with two types of corresponding DRR image information generated on the basis of a computed tomography (CT) image during a process of creating a treatment plan and generates, on the basis of the comparison results, positioning data that is necessary to position the couch 57. In addition, the positioning control device drives a positioning device (not shown) by using the positioning data so as to position the couch 57.

### Irradiation with beam

After the positioning of the patient 58 is completed, air is supplied into the rod-side cylinder room of the air cylinder 14 so that the air cylinder 14 pulls the X-ray detector 3 and positions the X-ray detector 3 at the retracted position. In addition, the X-ray tube 1 of the first X-ray imaging device is retracted from the beam axis m by a mechanism (not shown). Fig. 4 is a diagram showing the above state in which the X-ray detector 3 and the X-ray tube 1 are retracted.

After the retractions of the X-ray tube 1 (of the first X-ray imaging device) and the X-ray detector 3 are completed, the affected part of the patient 58 is irradiated with the charged particle beam and treated.

The operator such as a doctor sets an incident direction of the beam onto the affected part of the patient. This setting is performed by rotating the rotating gantry 54. Fig. 4 shows an example in which the beam propagates in the vertical direction and is incident on the affected part of the patient from the upper side. After the setting of the incident direction of the beam onto the affected part of the patient is completed, the charged particle beam is extracted from the synchrotron 62. The charged particle beam 19 propagates into the beam transport chamber 12 through the beam duct 17 and is transported in the beam transport chamber 12. After the charged particle beam 19 is deflected by the beam scanning magnets 11A and 11B, the affected part of the patient 58 is irradiated with the charged particle beam 19.

Normally, the affected part of the patient 58 is irradiated with the charged particle beam from at least two directions (multi-field irradiation). Specifically, after the affected part is irradiated with the charged particle beam from a certain direction, the rotating gantry 54 is rotated so that the orientation of the irradiation nozzle apparatus 55 is changed. Then, the affected part is irradiated with the charged particle beam from a direction that is different from the certain direction.

Next, effects of the present embodiment are described.

In the present embodiment, the X-ray tubes are arranged outside the irradiation nozzle apparatus 55, while those were arranged inside an irradiation nozzle apparatus in a conventional structure. Thus, while the length of the irradiation nozzle apparatus 55 is maintained equal to or nearly equal to that of the conventional one, the length of the beam transport chamber 12 that is included in the irradiation nozzle apparatus 55 can be increased and the diameter of the beam that passes through the irradiation nozzle apparatus 55 can be reduced, compared with the conventional irradiation nozzle apparatus.

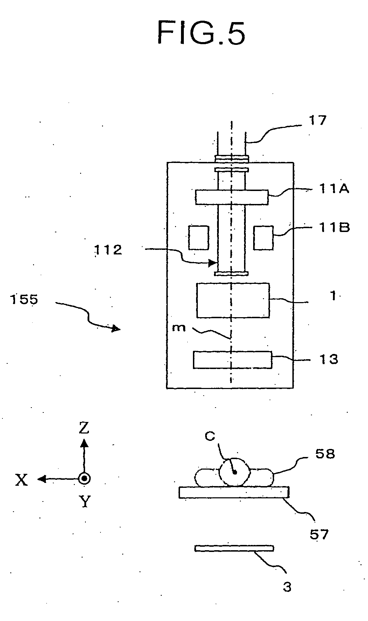

The effects are described below with reference to Fig. 5. Fig. 5 is a diagram showing, as a comparative example, a conventional irradiation nozzle apparatus that includes an X-ray tube and a beam transport chamber. In Fig. 5, the same members as in Fig. 3 are indicated by the same reference numerals in Fig. 3.

In Fig. 5, an X-ray tube 1 is arranged in an irradiation nozzle apparatus 155. As the X-ray tube 1 is relatively heavy and large, the position thereof in the irradiation nozzle apparatus 155 is limited. Thus, a space for installation of a beam transport chamber 112 in the irradiation nozzle apparatus 155 is limited due to the X-ray tube 1. Therefore, the length of the beam transport chamber 112 cannot be increased to a certain value or more, while the length of the irradiation nozzle apparatus 155 is maintained. As a result, a reduction in the diameter of a beam that passes through the irradiation nozzle apparatus 155 is limited.

In the present embodiment shown in Fig. 3, the X-ray tube is arranged outside the irradiation nozzle apparatus 55, and the X-ray detector is located in the irradiation nozzle apparatus 55. Thus, increasing in length of the beam transport chamber 12 is possible compared with the conventional beam transport chamber described in the comparative example shown in Fig. 5, while the length of the irradiation nozzle apparatus is maintained. If the length of the beam transport chamber 12 is increased, a distance in which the charged particle beam propagates while being exposed to air is shortened, and scattering of the charged particle beam is suppressed by the region in the beam transport chamber in which the gas is present. Therefore, this makes the size of a spot of the beam on the patient smaller than that in the comparative example.

Since the thickness of the X-ray detector 3 in the beam axis direction is smaller and the structure thereof is simpler compared with those of the X-ray tube 1, by arranging the X-ray detector 3 in the irradiation nozzle apparatus 55, it is possible to easily perform maintenance for the irradiation nozzle apparatus 55.

In addition, since the X-ray tube 1 is located outside the irradiation nozzle apparatus 55 that includes the housing 15 made of metal, it is possible to easily perform maintenance for the X-ray tube 1 and a device located on the side of the X-ray tube 1, such as replacement of the X-ray tube 1 with another X-ray tube.

In addition, since the X-ray tube 1 is arranged outside the irradiation nozzle apparatus 55, the irradiation nozzle apparatus 55 does not directly receive heat released by the X-ray tube 1, and whereby it is possible to prevent peripheral devices from being adversely thermally affected. Especially, since the beam dose monitor (beam monitor) 13 uses radiation-ionized gas, the beam dose monitor 13 is affected by the temperature of the gas. Therefore, if the X-ray tube 1 were arranged in the irradiation nozzle apparatus 55, the beam dose monitor 13 would be easily affected by heat released by the X-ray tube 1. Reducing the number of heat sources makes the temperature of the radiation-ionized gas stable, and effect to the accuracy of detecting the dose of the beam by heat released by the X-ray tube 1 is reduced. Therefore, the safety of the particle beam treatment apparatus can be improved.

In addition, since positions of the X-ray tube 1 and the X-ray detector 3 are only replaced with each other, an increase in the cost of the particle beam treatment apparatus can be suppressed, and a necessary imaging range can be ensured.

In addition, since the X-ray tube 1 is not attached to an exterior of the irradiation nozzle apparatus 55, this makes the operator such as a doctor efficiently perform a tooling for treatment without reducing the range of activities of the operator, when the rotating gantry 54 is in the home position.

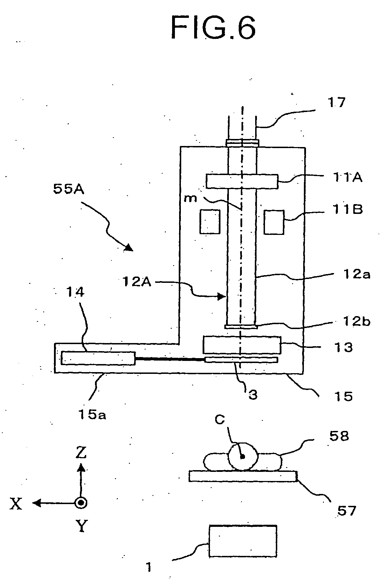

Fig. 6 is a diagram showing an irradiation nozzle apparatus according to another embodiment of the present invention. The irradiation nozzle apparatus shown in Fig. 6 is similar to that shown in Fig. 3 and includes a beam transport chamber that has a vacuum region instead of the gas region.

Referring to Fig. 6, the irradiation nozzle apparatus 55A according to the present embodiment includes a beam transport chamber 12A that has a vacuum region therein. An upstream-side end of the beam transport chamber 12A is connected to the beam duct 17.

According to the present embodiment, it is not necessary to install a window (isolated window 12c) through which the beam passes while air is blocked, at the upstream-side end of the beam transport chamber 12A. Thus, it is possible to further suppress scattering of the beam and reduce the diameter of the beam.

In the embodiments shown in Figs. 3, 4 and 6, the X-ray detector is moved in the rotational direction (X direction in Figs. 3, 4 and 6) of the rotating gantry and retracted. However, the X-ray detector may be moved in the direction (Y direction in Figs. 3, 4 and 6) of the rotational axis of the rotating gantry and retracted. In this case, since the X-ray detector is moved in the horizontal direction regardless of the angle of the rotation of the rotating gantry, this makes driving power be suppressed, and the accuracy of positioning of the X-ray detector at the inserted position easy to be improved.

## Claims

1. A particle beam treatment apparatus including:
a beam generator (51) adapted to accelerate a charged particle beam (19) to a set energy level;
an irradiation nozzle apparatus (55) that is arranged in a treatment room (59) and is adapted to form a field irradiated with the charged particle beam (19);
a beam transport unit (52) adapted to transport the charged particle beam (19) extracted from the beam generator (51) into the irradiation nozzle apparatus (55);
scanning magnets (11A, 11B) that are arranged in the irradiation nozzle apparatus (55) and adapted to deflect the charged particle beam (19) transported in the irradiation nozzle apparatus (55);
a beam transport chamber (12) that is arranged in the irradiation nozzle apparatus (55) so as to include a beam axis (m) and has a gas region or a vacuum region in order to suppress scattering of the charged particle beam (19);
**characterised by**:
an X-ray generator (1) that is arranged outside the irradiation nozzle apparatus (55) so as to be located on an opposite side of the irradiation nozzle apparatus (55) with respect to a couch (57); and
an X-ray detector (3) that is arranged in the irradiation nozzle apparatus (55) and is adapted to detect an X-ray beam emitted by the X-ray generator (1).

2. The particle beam treatment apparatus according to claim 1, wherein the X-ray detector (3) includes a flat panel detector.

3. The particle beam treatment apparatus according to claim 1, further comprising a beam monitor (13) that is arranged in the irradiation nozzle apparatus (55) and located on the downstream side of the scanning magnets (11A, 11 B) and the beam transport chamber (12) with respect to a direction in which the charged particle beam (19) propagates,
wherein the X-ray detector (3) is arranged in the irradiation nozzle apparatus (55) on a further downstream side of the beam monitor (13) so as to be retractable from the beam axis (m).

4. An irradiation nozzle apparatus (55) that is arranged in a treatment room (59) and is adapted to form a field irradiated with a charged particle beam (19) that has been extracted from a beam generator (51) and transported through a beam transport unit (52), the irradiation nozzle apparatus (55) comprising:
a housing (15);
scanning magnets (11A, 11B) that are arranged in the housing (15) and adapted to deflect the charged particle beam (19) transported in the irradiation nozzle apparatus (55);
a beam transport chamber (12) that is arranged in the housing (15) so as to include a beam axis (m) and has a gas region or a vacuum region in order to suppress scattering of the charged particle beam (19);
**characterised by** an X-ray detector (3) that is arranged in the housing (15) and is adapted to detect an X-ray beam emitted by an X-ray generator (1) that is arranged outside the housing (15).

5. The irradiation nozzle apparatus (55) according to claim 4, further comprising a beam monitor (13) that is arranged in the housing (15) so as to be located on a downstream side of the scanning magnets (11A, 11B) and the beam transport chamber (12) with respect to a direction in which the charged particle beam (19) propagates,
wherein the X-ray detector (3) is arranged in the housing (15) so as to be located on a further downstream side of the beam monitor (13) so that the X-ray detector (3) can be retracted from the beam axis (m).

## Patentansprüche

1. Teilchenstrahlbehandlungsgerät mit
einer Strahlerzeugungseinheit (51), die dazu ausgelegt ist, einen Ladungsteilchenstrahl (19) auf ein eingestelltes Energieniveau zu beschleunigen,
einem Bestrahlungskopfgerät (55), das in einem Behandlungsraum (59) angeordnet und dazu ausgelegt ist, ein mit dem Ladungsteilchenstrahl (19) bestrahltes Feld zu bilden,
einer Strahltransporteinheit (52), die dazu ausgelegt ist, den aus der Strahlerzeugungseinheit (51) extrahierten Ladungsteilchenstrahl (19) in das Bestrahlungskopfgerät (55) zu transportieren,
Abtastmagneten (11A, 11 B), die in dem Bestrahlungskopfgerät (55) angeordnet und dazu ausgelegt sind, den in das Bestrahlungskopfgerät (55) transportierten Ladungsteilchenstrahl (19) abzulenken,
einer Strahltransportkammer (12), die in dem Bestrahlungskopfgerät (55) so angeordnet ist, dass sie eine Strahlachse (m) einschließt und eine Gasregion oder eine Vakuumregion aufweist, um eine Streuung des Ladungsteilchenstrahls (19) zu unterdrücken,
**gekennzeichnet durch**
eine Röntgenstrahlerzeugungseinheit (1), die außerhalb des Bestrahlungskopfgeräts (55) angeordnet ist, so dass sie auf einer bezüglich einer Liege (57) entgegengesetzten Seite des Bestrahlungskopfgeräts (55) positioniert ist, und
einen Röntgenstrahldetektor (3), der in dem Bestrahlungskopfgerät (55) angeordnet und dazu ausgelegt ist, einen aus der Röntgenstrahlerzeugungseinheit (1) emittierten Röntgenstrahl zu erfassen.

2. Teilchenstrahlbehandlungsgerät nach Anspruch 1, wobei der Röntgenstrahldetektor (3) einen Flachdetektor aufweist.

3. Teilchenstrahlbehandlungsgerät nach Anspruch 1, ferner mit einem Strahlmonitor (13), der in dem Bestrahlungskopfgerät (55) angeordnet und auf der bezüglich einer Richtung, in der der Ladungsteilchenstrahl (19) sich ausbreitet, strahlabwärtigen Seite der Abtastmagnete (11A, 11B) und der Strahltransportkammer (12) positioniert ist,
wobei der Röntgenstrahldetektor (3) in dem Bestrahlungskopfgerät (55) auf einer weiter strahlabwärtigen Seite des Strahlmonitors (13) angeordnet ist, so dass er aus der Strahlachse (m) wegziehbar ist.

4. Bestrahlungskopfgerät (55), das in einem Behandlungsraum (59) angeordnet und dazu ausgelegt ist, ein Feld zu bilden, das mit einem Ladungsteilchenstrahl (19) bestrahlt wird, der aus einer Strahlerzeugungseinheit (51) extrahiert und durch eine Strahltransporteinheit (52) transportiert wurde, wobei das Bestrahlungskopfgerät (55) aufweist:
ein Gehäuse (15),
Abtastmagnete (11A, 11B), die in dem Gehäuse (15) angeordnet und dazu ausgelegt sind, den in das Bestrahlungskopfgerät (55) transportierten Ladungsteilchenstrahl (19) abzulenken,
eine Strahltransportkammer (12), die in dem Gehäuse (15) so angeordnet ist, dass sie eine Strahlachse (m) einschließt und eine Gasregion oder eine Vakuumregion aufweist, um eine Streuung des Ladungsteilchenstrahls (19) zu unterdrücken,
**gekennzeichnet durch** einen Röntgenstrahldetektor (3), der in dem Gehäuse (15) angeordnet und dazu ausgelegt ist, einen Röntgenstrahl zu erfassen, der aus einer außerhalb des Gehäuses (15) angeordneten Röntgenstrahlerzeugungseinheit (1) emittiert wurde.

5. Bestrahlungskopfgerät (55) nach Anspruch 4, ferner mit einem Strahlmonitor (13), der in dem Gehäuse (15) angeordnet ist, so dass er auf einer bezüglich einer Richtung, in der sich der Ladungsteilchenstrahl (19) ausbreitet, strahlabwärtigen Seite der Abtastmagnete (11A, 11B) und der Strahltransportkammer (12) positioniert ist,
wobei der Röntgenstrahldetektor (3) in dem Gehäuse so angeordnet ist, dass er auf einer weiter strahlabwärtigen Seite des Strahlmonitors (13) positioniert ist, so dass der Röntgenstrahldetektor (3) aus der Strahlachse (m) wegziehbar ist.

## Revendications

1. Appareil de traitement de faisceau de particules comprenant :
un générateur de faisceau (51) adapté pour accélérer un faisceau de particules chargées (19) jusqu'à un niveau d'énergie établi,
un appareil à buse d'irradiation (55) qui est disposé dans un espace de traitement (59) et est adapté pour former un champ irradié à l'aide du faisceau de particules chargées (19),
une unité de transport de faisceau (52) adaptée pour transporter le faisceau de particules chargées (19) extrait du générateur de faisceau (51) dans l'appareil à buse d'irradiation (55),
des aimants de balayage (11A, 11B) qui sont disposés dans l'appareil à buse d'irradiation (55) et adaptés pour dévier le faisceau de particules chargées (19) transporté dans l'appareil à buse d'irradiation (55),
une chambre de transport de faisceau (12) qui est disposée dans l'appareil à buse d'irradiation (55) de manière à inclure un axe du faisceau (m) et dispose d'une région gazeuse ou d'une région sous vide afin de supprimer la diffusion du faisceau de particules chargées (19),
**caractérisé par** :
un générateur de rayons X (1) qui est disposé à l'extérieur de l'appareil à buse d'irradiation (55) de manière à être positionné sur un côté opposé de l'appareil à buse d'irradiation (55) par rapport à un support (57), et
un détecteur de rayons X (3) qui est disposé dans l'appareil à buse d'irradiation (55) et est adapté pour détecter un faisceau de rayons X émis par le générateur de rayons X (1).

2. Appareil de traitement de faisceau de particules selon la revendication 1, dans lequel le détecteur de rayons X (3) comprend un détecteur à écran plat.

3. Appareil de traitement de faisceau de particules selon la revendication 1, comportant en outre un moniteur de faisceau (13) qui est disposé dans l'appareil à buse d'irradiation (55) et positionné sur le côté aval des aimants de balayage (11A, 11B) et de la chambre de transport de faisceau (12) par rapport à une direction dans laquelle le faisceau de particules chargées (19) se propage,
dans lequel le détecteur de rayons X (3) est disposé dans l'appareil à buse d'irradiation (55) sur un côté aval supplémentaire du moniteur de faisceau (13) de manière à pouvoir être rétracté de l'axe du faisceau (m).

4. Appareil à buse d'irradiation (55) qui est disposé dans un espace de traitement (59) et est adapté pour former un champ irradié à l'aide d'un faisceau de particules chargées (19) qui a été extrait d'un générateur de faisceau (51) et transporté à travers une unité de transport de faisceau (52), l'appareil à buse d'irradiation (55) comportant :
un boîtier (15),
des aimants de balayage (11A, 11B) qui sont disposés dans le boîtier (15) et adaptés pour dévier le faisceau de particules chargées (19) transporté dans l'appareil à buse d'irradiation (55),
une chambre de transport de faisceau (12) qui est disposée dans le boîtier (15) de manière à inclure un axe du faisceau (m) et présente une région gazeuse ou une région sous vide afin de supprimer la diffusion du faisceau de particules chargées (19),
**caractérisé par** un détecteur de rayons X (3) qui est disposé dans le boîtier (15) et est adapté pour détecter un faisceau de rayons X émis par un générateur de rayons X (1) qui est disposé à l'extérieur du boîtier (15)

5. Appareil à buse d'irradiation (55) selon la revendication 4, comportant en outre un moniteur de faisceau (13) qui est disposé dans le boîtier (15) de manière à être positionné sur un côté aval des aimants de balayage (11A, 11B) et de la chambre de transport de faisceau (12) par rapport à une direction dans laquelle le faisceau de particules chargées (19) se propage,
dans lequel le détecteur de rayons X (3) est disposé dans le boîtier (15) de manière à être positionné sur un côté aval supplémentaire du moniteur de faisceau (13) de sorte que le détecteur de rayons X (3) peut être rétracté de l'axe du faisceau (m).
